Europäisches Patentamt

European Patent Office

Office européen des brevets

(11) Publication number: **0 333 683**
**A1**

## (12) EUROPEAN PATENT APPLICATION

(21) Application number: **89850069.9**

(22) Date of filing: **24.02.89**

(51) Int. Cl.⁴: **A 61 N 5/10**

(30) Priority: **02.03.88 SE 8800740**

(43) Date of publication of application:
**20.09.89 Bulletin 89/38**

(84) Designated Contracting States:
**AT CH DE FR GB LI NL SE**

(71) Applicant: **Denev, Martin Ivanov**
**Box 214**
**S-133 02 Saltsjöbaden (SE)**

(72) Inventor: **Denev, Martin Ivanov**
**Box 214**
**S-133 02 Saltsjöbaden (SE)**

The title of the invention has been amended (Guidelines for Examination in the EPO, A-III, 7.3).

(54) **Radiation treatment of cancer tumours applied to the cancerous antimatter.**

㋝ The invention belongs to the technical area of medical technics and devices for radiation treatment of cancer tumours.

At radiation treatment of cancercells one is destroying not only the cancer tumours but also other parts of the body and therefore there exists problem with creating methods and devices that will only destroy the cancercells and not damage the rest of the body.

As contribution to the solution of this problem is suggested this method and device.

By physics experiment, awarded with Nobel prize in physics, is known that according to the laws of symmetry matter situated before a mirror gets a double of antimatter at the same distance from the mirror (which is symmetry axis) behind the mirror.

The theory of the inventor is that if one by radiation destroys the antitumour (the double) which is of antimatter and is situated behind the mirror, one will because of the symmetry also destroy the cancer tumour which is of matter and is situated in the body at the exact distance from the mirror as its double.

With the description is illustrated the idea of the invention; The patient 1, with the cancer tumour 2, is fixed at a table 3 before the mirror 5. At the same distance from the mirror 4 is the double of the cancer tumour 2A which is irradiated by the beam 5 after that the computer 6 has estimated the exact symmetric location for irradiation behind the mirror.

EP 0 333 683 A1

## Description

## Method and device for radiation treatment of cancer tumours with applying of the beam outside of the body, to the symmetric antimatter of the cancer tumour which arises behind a mirror.

At radiation treatment of cancercells one is destroying not only the cancer tumours but also other parts of the body and therefore there exists problem with creating methods and devices that will only destroy the cancercells and not damage the rest of the body. As contribution to the solution of this problem is suggested this method and device.

By physics experiment, awarded with Nobel prize in physics, is known that according to the laws of symmetry matter situated before a mirror gets a double of antimatter at the same distance from the mirror (which is a symmetry axis) behind the mirror. The theory of the inventor is that if one by radiation destroys the antitumor (the double), which is of antimatter and is situated behind the mirror, one will because fo the symmetry also destroy the cancer tumour which is of matter and is situated iin the body at the exact distance from the mirror as its double.

With the description is illustrated the idea of the invention: The patient 1, with the tumour 2, is fixed at a table 3, before the mirror 4. At the same distance from the mirror 4 is the double of the cancer tumour 2A which is irradiated by the beam 5 after that the computer 6 has estimated the exact symmetric location for irradiation behind the mirror.

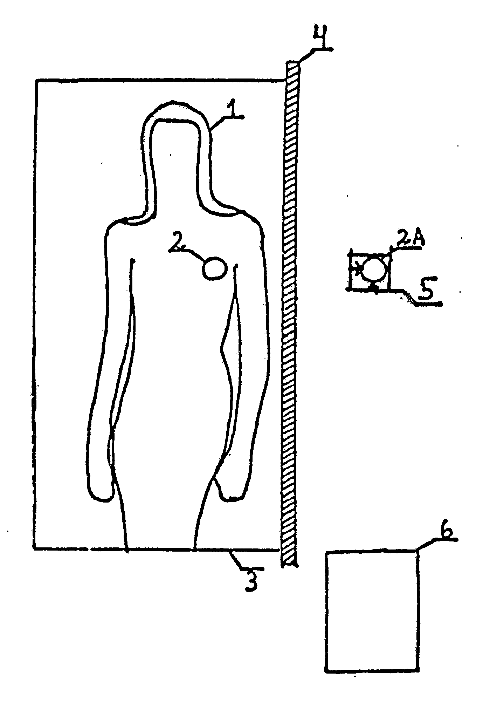

## Claims

Device for radiation treatment of cancer tumours with applying of the beam outside of the body, to the symmetric antimatter of the cancer tumour which arises behind a mirror, characterized by that the patient 1, with the cancer tumour 2, is fixed at the table 3 before the mirror 4 and the beam 5 is applied to the double-cancer tumour 2A after that the location for irradiation has beed estimated by the computer 6.

European Patent Office

**EUROPEAN SEARCH REPORT**

Application Number

EP 89 85 0069

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | -A-       (No relevant documents have been disclosed) ----- | | A 61 N   5/10 |

|  |
|---|
| TECHNICAL FIELDS SEARCHED (Int. Cl.4) |
| A 61 N |

The present search report has been drawn up for all claims

| Place of search | Date of completion of the search | Examiner |
|---|---|---|
| THE HAGUE | 07-06-1989 | LEMERCIER D.L.L. |

EPO FORM 1503 03.82 (P0401)